# EUROPÄISCHE PATENTSCHRIFT

(11) **EP 0 474 996 B1**
(45) Veröffentlichungstag und Bekanntmachung des Hinweises auf die Patenterteilung: **20.10.1993**
(21) Anmeldenummer: 91110720.9
(22) Anmeldetag: 28.06.1991
(51) Int. Cl.: C07C 67/08, C07C 69/24

(54) **Verfahren zur Herstellung von Estern**
Process for preparing esters
Procédé de préparation d'esters

(30) Priorität: 31.08.1990 DE 4027639
(43) Veröffentlichungstag der Anmeldung: 18.03.1992
(73) Patentinhaber: HÜLS AKTIENGESELLSCHAFT, 45764 Marl (DE)
(72) Erfinder: Kahsnitz, John Dr., W-4358 Haltern (DE); Oberholz, Alfred, Dr., W-4370 Marl (DE); Knippenberg, Udo, W-4358 Haltern-Lippramsdorf (DE); Zölffel, Michael, W-4370 Marl (DE)

(56) Entgegenhaltungen:
- WO-A-90/08127
- WO-A-91/01966
- DE-A- 1 768 104
- DE-A- 3 121 383
- GB-A- 1 387 704
- CHEMIE. INGENIEUR. TECHNIK. Bd. 54, Nr. 2, 1982, WEINHEIM DE Seite 163; H. SCHOENMAKERS: 'Destillationskolonne mit aussenliegenden Reaktoren'

## Beschreibung

Die Erfindung betrifft ein Verfahren zur Herstellung von Estern aus Alkoholen mit 1 bis 6 Kohlenstoffatomen und Carbonsäuren mit 6 bis 20 Kohlenstoffatomen durch flüssigphasige Gleichgewichtsreaktionen an Ionenaustauschern in einem Vorreaktor und in außenliegenden Reaktoren einer Rektifikationskolonne, wobei das Reaktionsprodukt in der Kolonne aufgetrennt wird.

Da die Veresterung eine nicht sehr einseitige Gleichgewichtsreaktion ist, werden besondere Maßnahmen, wie zum Beispiel der Einsatz einer Ausgangsverbindung im Überschuß oder eine mehrstufige Reaktionsführung, ergriffen, um den Umsatz wertvoller Einsatzstoffe zu erhöhen und um die Ester in möglichst hoher Ausbeute und Reinheit zu erhalten.

So wird die Veresterung an Kationentauschern nach DE-C-1 768 104 in einer Vorkolonne und einer nachgeschalteten Reaktionsdestillationskolonne durchgeführt.

Wegen der hohen thermischen Belastung von Ionenaustauschern in einer Reaktionsdestillationskolonne und wegen der schwierigen destillativen Trennung in Gegenwart von Ionenaustauschern werden bei anderen Verfahren Veresterung und Rektifikation räumlich getrennt. In DE-C-31 21 383 wird in einem Reaktor an einem Kationenaustauscher verestert. In einer nachgeschalteten kationenaustauscherfreien Destillationskolonne wird das Reaktionsgemisch aufgetrennt. Durch Rückführung unumgesetzter Ausgangsverbindungen in den Reaktor können hier hohe Esterausbeuten erzielt werden.

H. Schoenmakers und W. Bühler, Chem.-Ing.-Tech. 54 (1982), 163, weisen auf eine weitere Möglichkeit der Veresterung hin. Sie verwenden eine Apparatur, die aus einem Vorreaktor und aus einer Destillationskolonne mit außenliegenden Reaktoren besteht. Die Veresterung wird in den Reaktoren durch Ionenaustauscher katalysiert. Dabei wird das Alkohol-Säure-Gemisch zuerst in den Vorreaktor geführt. Das Reaktionsgemisch wird auf die Destillationskolonne gegeben. Die in der Kolonne abfließende Flüssigphase wird mehrmals seitlich herausgeführt, durch einen außenliegenden Reaktor geleitet und von dort auf den nächsttieferen Boden der Kolonne zurückgegeben.

Bei äquimolaren Mengen an Alkohol und Säure oder bei leicht überschüssigen Alkoholmengen werden Umsätze von etwa 55 bis 60 % erzielt. Das Verfahren vermeidet zwar die Rückführung unumgesetzter Ausgangsverbindungen in den Vorreaktor, die Umsätze sind aber für eine kommerzielle Verwertung sehr gering.

Aufgabe der vorliegenden Erfindung war es deshalb, bei der Veresterung in einer Apparatur aus einem Vorreaktor und einer Rektifikationskolonne mit außenliegenden Reaktoren, bei der man der Kolonne jeweils auf einem Boden Flüssigphase seitlich abzieht, einem außenliegenden Reaktor zuführt und von dort auf den nächsttieferen Boden der Kolonne zurückgibt, Umsatz und Ausbeute zu verbessern.

Die Aufgabe wird erfindungsgemäß dadurch gelöst, daß man eine Ausgangsverbindung zusätzlich den außenliegenden Reaktoren direkt zuführt.

Ausgangsverbindungen im Sinne der vorliegenden Erfindung sind Alkohole mit 1 bis 6 Kohlenstoffatomen und Carbonsäuren mit 6 bis 20 Kohlenstoffatomen. Sie werden vorzugsweise in solchen Mengen in den Vorreaktor eingesetzt, daß auf ein Äquivalent Carbonsäure 0,2 bis 6 Äquivalente Alkohol kommen. Dabei wird meist die wertvollere Komponente im Unterschuß eingesetzt.

Geeignete Carbonsäuren sind beispielsweise Hexan-, Decan-, Dodecan- und Tetradecansäure, Palmitin-, Linol- und Stearinsäure sowie Benzoesäure. Auch mehrwertige Säuren wie Adipinsäure, Dodecandisäure, Citronensäure oder Isophthalsäure sind einsetzbar. Aliphatische Monocarbonsäuren mit 10 bis 16 Kohlenstoffatomen werden bevorzugt eingesetzt.

Geeignete Alkohole sind beispielsweise Methanol, Ethanol, Propanol, Isopropanol, Butanol, Pentanol und Hexanol sowie Cyclohexanol. Bevorzugt werden Methanol und Ethanol verwendet.

In einer besonderen Ausführungsform der Erfindung werden auf 1 Äquivalent Carbonsäure 1 bis 5 Äquivalente Alkohol in den Vorreaktor eingesetzt.

Als Ionenaustauscher werden handelsübliche saure Kationenaustauscher verwendet.

Vorzugsweise setzt man Rektifikationskolonnen mit 5 bis 30 theoretischen Böden ein. An einer Rektifikationskolonne sind vorzugsweise 2 bis 5 außenliegende Reaktoren angeschlossen.

In die außenliegenden Reaktoren wird vorzugsweise die gesamte herunterfließende Flüssigphase geleitet. Wird nur ein Teil der Flüssigphase in die außenliegenden Reaktoren abgeleitet, so wird ein geringerer Umsatz erzielt.

Die Ausgangsverbindung, die in die außenliegenden Reaktoren zudosiert wird, ist vorzugsweise die Verbindung, die im Vorreaktor in äquivalenter Menge oder im Überschuß eingesetzt wurde. Da die Carbonsäure im vorliegenden Verfahren vorzugsweise die wertvollere Verbindung ist und deshalb meist in äquivalenter Menge oder im Unterschuß eingesetzt wird, wird Alkohol vorzugsweise in den außenliegenden Reaktoren zugegeben.

In einer besonders bevorzugten Ausführungsform werden in jeden außenliegenden Reaktor pro Äquivalent eingesetzter Carbonsäure 0,2 bis 5 Äquivalente Alkohol gegeben.

Besonders vorteilhaft ist es, die Mischung, die die außenliegenden Reaktoren verläßt, teilweise zu verdampfen. Vorzugsweise werden dabei bis zu 85 Mol-% der Mischung verdampft.

Die verbleibende Flüssigphase wird dann wie bisher auf den ersten Boden unterhalb der Entnahmestelle zurückgeführt. Die Dampfphase wird aber gesondert in die Kolonne geleitet, und zwar auf den ersten Boden oberhalb der Entnahmestelle.

Eine Teilverdampfung bietet sich vor allem dann an, wenn die Reaktoren unter einem höheren Druck stehen als die Kolonnne. Die Teilverdampfung kann beispielsweise mit Hilfe eines Flash-Verdampfers erfolgen. In einer sehr bevorzugten Fahrweise werden 40 bis 80 Mol-% der Mischung verdampft.

Wegen der begrenzten thermischen Stabilität der sauren Ionenaustauscher wird die Veresterung im Vorreaktor und in den außenliegenden Reaktoren bei Temperaturen bis etwa 120 °C durchgeführt. Dabei werden Temperaturen von 40 bis 100 °C vorzugsweise eingestellt.

In der Rektifikationskolonne erfolgt die Auftrennung der Reaktionsprodukte vorzugsweise bei Normaldruck. Es können aber auch Drücke bis etwa 0,8 MPa angewandt werden.

In den Reaktoren kann ein höherer Druck als in der Kolonne von Vorteil sein. Im allgemeinen liegt der Druck in den Reaktoren im Bereich von Normaldruck bis etwa 1 MPa.

Nach dem erfindungsgemäßen Verfahren wird der Umsatz an Unterschußverbindung erhöht. Die Ausbeute an Ester wird deutlich verbessert. Es werden Ester mit verminderter Säurezahl hergestellt.

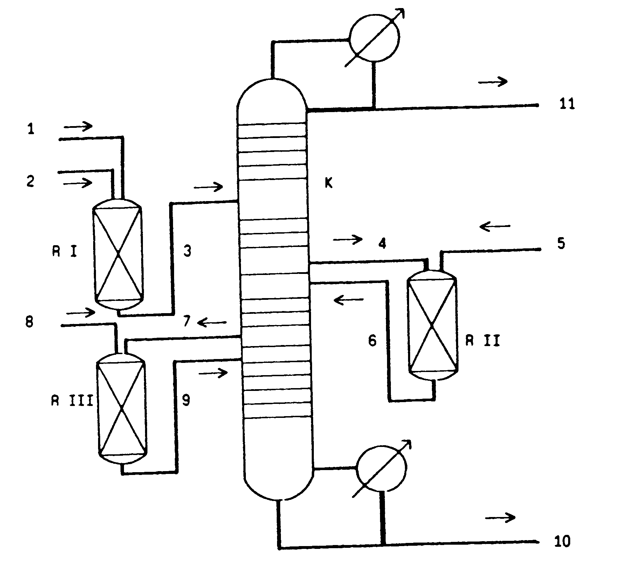

Abbildung 1 zeigt schematisch eine Apparatur aus einem Vorreaktor R1 und einer Rektifikationskolonne K mit den außenliegenden Reaktoren R II und R III. Bei einer Umsetzung, bei der Alkohol den außenliegenden Reaktoren zudosiert und bei der keine Teilverdampfung vorgenommen wird, wird wie folgt verfahren:

Die Carbonsäure wird über Leitung 1, der Alkohol über Leitung 2 in den Reaktor R I geführt. Das Reaktionsprodukt gelangt über Leitung 3 in die Kolonne K. Über Leitung 4 wird die Flüssigphase in den Reaktor R II geleitet, über Leitung 5 wird Alkohol zudosiert. Das Reaktionsprodukt wird über Leitung 6 zurückgeführt. Über Leitung 7 wird Flüssigphase erneut herausgeleitet. Durch Leitung 8 wird Alkohol geführt. Aus dem Reaktor R III gelangt das Produkt über Leitung 9 in die Kolonne zurück. Ester und restliche Carbonsäure werden über Leitung 10 ausgeführt, Destillate, Alkohol und Wasser, werden über Leitung 11 abgeleitet.

In den Beispielen 1 bis 3 und in Vergleichsbeispiel A wird gemäß Abbildung 1 eine bei Normaldruck betriebene, beheizte Rektifikationskolonne mit einem Innendurchmesser von dᵢ = 80 mm und mit 18 praktischen Böden verwendet. Die 3 Reaktoren sind baugleich und enthalten insgesamt 2,9 kg sauren Ionenaustauscher (LEWATIT^{R} SPC 108, Fa. Bayer AG, D-5090 Leverkusen). Am Kopf der Kolonne beträgt die Temperatur 70 °C, in der Blase 150 °C.

Die Temperatur der Reaktoren beträgt
- T_{RI}: = 66,0 °C
- T_{RII}: = 55,0 °C
- T_{RIII}: = 63,5 °C

### Vergleichsbeispiel A (nach H. Schoenmakers)

In den Reaktor R I werden 6,03 mol/h Dodecansäure und 18,24 mol/h Methanol gefahren. Den beiden außenliegenden Reaktoren wird ausschließlich abgezogene Flüssigphase aus der Kolonne zugeführt. Es wird kein weiteres Methanol zugegeben.
- Gesamtumsatz:: 70,3 %, bezogen auf Dodecansäure
- Gesamtenergieverbrauch:: 311 W

### Beispiel 1

In den Reaktor R I werden 6,03 mol/h Dodecansäure und 6,03 mol/h Methanol gefahren. Den beiden außenliegenden Reaktoren werden neben abgezogener Flüssigphase auch Methanol zugeführt: 6,42 mol/h Methanol für R II und 5,39 mol/h Methanol für R III.
- Gesamtumsatz:: 82,8 %, bezogen auf Dodecansäure
- Gesamtenergieverbrauch:: 305 W

Vergleichsbeispiel A und Beispiel 1 zeigen, daß unter gleichen Reaktionsbedingungen bei gleicher Säuremenge und fast gleicher Alkoholgesamtmenge sowie bei vergleichbarem Energieverbrauch nach dem erfindungsgemäßen Verfahren ein um 12,5 % höherer Umsatz erzielt wird.

### Beispiel 2

In den Reaktor R I werden 4,14 mol/h Dodecansäure und 12,34 mol/h Methanol gefahren. Den beiden außenliegenden Reaktoren werden neben abgezogener Flüssigphase auch Methanol zugeführt: 12,65 mol/h Methanol für R II und 12,96 mol/h Methanol für R III.
- Gesamtumsatz:: 99,5 %, bezogen auf Dodecansäure
- Gesamtenergieverbrauch:: 425 W

### Beispiel 3

In den Reaktor R I werden 6,03 mol/h Dodecansäure und 17,98 mol/h Methanol gefahren. Den beiden außenliegenden Reaktoren werden neben abgezogener Flüssigphase auch Methanol zugeführt: 17,98 mol/h Methanol für R II und 17,98 mol/h Methanol für R III. Der Reaktor R II wird jedoch abweichend von der obengenannten Fahrweise bei 0,5 MPa und 100 °C betrieben. Das Produkt des Reaktors R II wird in einem nachgeschalteten Flash-Verdampfer entspannt. 10,5 mol/h Flüssigphase werden dem ersten Boden unterhalb der Entnahmestelle zugeführt. 32,3 mol/h Dampfphase werden gesondert auf den ersten Boden über der Entnahmestelle der Kolonne geleitet.
- Gesamtumsatz:: 99,7
- Gesamtenergieverbrauch:: 300 W + Verdichtungsleistung vor R II

## Patentansprüche

1. Verfahren zur Herstellung von Estern aus Alkoholen mit 1 bis 6 Kohlenstoffatomen und Carbonsäuren mit 6 bis 20 Kohlenstoffatomen durch flüssigphasige Gleichgewichtsreaktionen an Ionenaustauschern in einem Vorreaktor (R I) und in außenliegenden Reaktoren (R II und R III) einer Rektifikationskolonne (K) sowie durch Auftrennung der Reaktionsprodukte in der Rektifikationskolonne (K), wobei man Flüssigphase auf einem Boden der Rektifikationskolonne (K) entnimmt, einem außenliegenden Reaktor zuführt und von dort auf den nächst tieferen Boden der Kolonne (K) zurückführt,
dadurch gekennzeichnet,
daß man zusätzlich eine Ausgangsverbindung den außenliegenden Reaktoren (R II und R III) direkt zuführt.

2. Verfahren nach Anspruch 1,
dadurch gekennzeichnet,
daß man auf 1 Äquivalent Carbonsäure 0,2 bis 6 Äquivalente Alkohol in den Vorreaktor (R I) einleitet.

3. Verfahren nach Anspruch 1,
dadurch gekennzeichnet,
daß man auf 1 Äquivalent Carbonsäure 1 bis 5 Äquivalente Alkohol in den Vorreaktor (R I) einsetzt und daß man in jeden außenliegenden Reaktor (R II und R III) pro Äquivalent ursprünglich eingesetzter Carbonsäure 0,2 bis 5 Äquivalente Alkohol gibt.

4. Verfahren nach Anspruch 1,
dadurch gekennzeichnet,
daß man Carbonsäuren mit 10 bis 16 Kohlenstoffatomen und Methanol oder Ethanol verwendet.

5. Verfahren nach Anspruch 1,
dadurch gekennzeichnet,
daß man die Reaktionsmischung, die die außenliegenden Reaktoren (R II und R III) verläßt, teilverdampft und dann die verbleibende Flüssigphase auf den nächst tieferen Boden und die gebildete Gasphase auf den nächst höheren Boden der Kolonne (K), bezogen auf die Entnahmestelle, zurückführt.

6. Verfahren nach Anspruch 5,
dadurch gekennzeichnet,
daß man bis zu 85 Mol-% der Reaktionsmischung verdampft.

7. Verfahren nach Anspruch 1,
dadurch gekennzeichnet,
daß man eine Rektifikationskolonne (K) mit 2 bis 5 außenliegenden Reaktoren (R II und R III) einsetzt.

## Claims

1. A process for the preparation of esters from alcohols having 1 to 6 carbon atoms and carboxylic acids having 6 to 20 carbon atoms by liquid-phase equilibrium reactions on ion exchangers in a pre-reactor (R I) and in external reactors (R II) and (R III) of a rectification column (K) and by separating out the reaction products in the rectification column (K), where liquid phase is withdrawn from a tray of the rectification column (K), fed to an external reactor and returned from there to the next lower tray of the column (K),
characterised in that a starting compound is additionally directly fed to the external reactors (R II and R III).

2. A process according to Claim 1,
characterised in that, per equivalent of carboxylic acid, 0.2 to 6 equivalents of alcohol are passed into the prereactor (R I).

3. A process according to Claim 1,
characterised in that, per equivalent of carboxylic acid, 1 to 5 equivalents of alcohol are used in the pre-reactor (R I) and in that, per equivalent of carboxylic acid originally used, 0.2 to 5 equivalents of alcohol are added to each external reactor (R II and R III).

4. A process according to Claim 1,
characterised in that carboxylic acids having 10 to 16 carbon atoms and methanol or ethanol are used.

5. A process according to Claim 1,
characterised in that the reaction mixture which leaves the external reactors (R II and R III) is evaporated in part and then the remaining liquid phase is returned to the next lower tray and the gas phase formed is returned to the next higher tray of the column (K), based on the withdrawal point.

6. A process according to Claim 5,
characterised in that up to 85 molar percent of the reaction mixture is evaporated.

7. A process according to Claim 1,
characterised in that a rectification column (K) having 2 to 5 external reactors (R II and R III) is used.

## Revendications

1. Procédé de préparation d'esters à partir d'alcools comportant de 1 à 6 atomes de carbone et d'acides carboxyliques comportant de 6 à 20 atomes de carbone, par des réactions d'équilibre en phase liquide sur des échangeurs d'ions, dans un pré-réacteur (R I) et dans des réacteurs situés à l'extérieur (R II et R III) d'une colonne (K) de rectification, ainsi que par séparation des produits réactionnels dans la colonne (K) de rectification, tandis que l'on prélève la phase liquide sur un plateau de la colonne (K) de rectification, qu'on la délivre à un réacteur situé à l'extérieur et qu'on la renvoie de là au plateau immédiatement inférieur de la colonne (K),
caractérisé par le fait que complémentairement on délivre directement un composé de départ aux réacteurs (R II et R III) situés à l'extérieur.

2. Procédé selon la revendication 1,
caractérisé par le fait que l'on introduit dans le pré-réacteur (R I) de 0,2 à 6 équivalents d'alcool par équivalent d'acide carboxylique.

3. Procédé selon la revendication 1,
caractérisé par le fait que dans le pré-réacteur (R I), on utilise de 1 à 5 équivalents d'alcool par équivalent d'acide carboxylique et que, dans chaque réacteur (R II et R III) se trouvant à l'extérieur, on ajoute de 0,2 à 5 équivalents d'alcool par équivalent d'acide carboxylique initialement mis en oeuvre.

4. Procédé selon la revendication 1,
caractérisé par le fait que l'on utilise des acides carboxyliques comportant de 10 à 16 atomes de carbone et du méthanol ou de l'éthanol.

5. Procédé selon la revendication 1,
caractérisé par le fait que l'on évapore partiellement le mélange réactionnel quittant les réacteurs (R II et R III) se trouvant à l'extérieur et que l'on renvoie ensuite la phase liquide qui reste sur le plateau immédiatement inférieur et que l'on renvoie la phase gazeuse formée sur le plateau immédiatement supérieur de la colonne (K), relativement au point de prélèvement.

6. Procédé selon la revendication 5,
caractérisé par le fait que l'on évapore jusqu'à 85 mol.-% du mélange réactionnel.

7. Procédé selon la revendication 1,
caractérisé par le fait que l'on utilise une colonne de rectification (K) à l'extérieur de laquelle se trouvent de 2 à 5 réacteurs (R II et R III).
